# EUROPEAN PATENT APPLICATION

(11) **EP 1 715 697 A2**
(43) Date of publication of application: **25.10.2006**
(21) Application number: 06112552.2
(22) Date of filing: 12.04.2006
(51) Int. Cl.: H04N 9/04

(54) **Color filter array with blue elements**

(30) Priority: 18.04.2005 US 107970
(71) Applicant: Given Imaging Ltd., Yoqneam Ilite 20692 (IL)
(72) Inventor: Horn, Eli, Kiryat Motzkin 26315 (IL); Iddan, Gavriel J., Haifa 34602 (IL)
(74) Representative: Dr. Graf & Partner

(57) **Abstract**

A color filter array for use in an imaging device including for example red sensitive elements, blue sensitive elements and green sensitive elements, where such blue sensitive elements may occur at a frequency of approximately twice that of said red sensitive elements and said green sensitive elements.

## Description

### FIELD OF THE INVENTION

The present invention relates to solid state image sensors and to color filters that permit light of a particular color to reach a photo-sensor or imager and to in-vivo imaging devices including them.

### BACKGROUND OF THE INVENTION

Solid state imaging cells may include an array of pixel cells containing for example photosensors such as for example phototransistors, photoconductors or photodiodes. To detect color, a filter may be placed in front of a photosensor, so that the photosensor may detect only the color that may be permitted to pass through the portion of the filter that may be in front of, or covering, the photosensor. Such a filter may be known as a color filter array (CFA). Typically, the colors represented in a CFA may be red, blue and green, and the colored filters may be arranged in a Bayer pattern such that green filters may be present with a frequency of approximately twice that of red filters and/or of blue filters. Such frequency may be effective due to the heightened sensitivity of green to the human eye, and may be appropriate for imaging objects having a fairly uniform representation of colors across a visible spectrum. The Bayer color filter pattern may be commonly used in imagers, for example, CMOS imagers and may be optimized to the real world which may be rich in green components. The Bayer color filter is designed so that for every red or blue pixel, two green pixels are acquired. This may enable construction of a relatively high resolution green plane image, while red and blue images may be reconstructed in a slightly reduced resolution. Additional colors other than red and green may be used.

Alternate CFA patterns may be known for use in imaging objects in-vivo that may also not have a uniform representation of colors across the visible spectrum. For example, a CFA designed for in-vivo imaging is known with a relatively high resolution red plane image, such that for every blue or green pixel, two red pixels may be acquired. The motivation for increasing the resolution of the red plane in this example may be due to a predominance increase, or relative larger number of red-colored objects that may exist in in-vivo environments.

However, red light, having a longer wavelength than blue may also penetrate the tissue before being reflected. Images of such objects may therefore appear blurred or lacking in sharpness as red light may be reflected back from various layers of tissue.

### SUMMARY OF THE INVENTION

In some embodiments of the present invention, an in-vivo imaging device may include CFA, the CFA including red sensitive elements, blue sensitive elements, and green sensitive elements, the blue sensitive elements occurring in the array substantially more frequently than said red sensitive elements and said blue sensitive elements occurring in the array substantially more frequently than said green sensitive elements. In one embodiment of the present invention, the blue sensitive elements occur approximately twice as frequently as the red sensitive elements and approximately twice as frequently as the green sensitive elements.

### BRIEF DESCRIPTION OF THE DRAWINGS

The present invention will be understood and appreciated more fully from the following detailed description taken in conjunction with the drawings in which:
Figure 1 is schematic drawing showing the penetration of blue wavelength through tissue as compared to red wavelength, in accordance with embodiments of the present invention;
Figure 2A is a schematic drawing of a color filter array pattern having a predominance or larger number of blue elements in accordance with embodiments of the invention;
Figure 2B is an exploded view of a color filter array in accordance with an embodiment of the invention;
Figure 3 is a schematic diagram of elements of a solid state imager having a color filter array in accordance with an embodiment of the invention;
Figure 4 is a schematic diagram of a system including an imager with a color filter pattern in accordance with an embodiment of the invention;
Figure 5 is a schematic drawing of a color filter array according to another embodiment of the present invention; and
Figure 6 is a flow diagram of a method of receiving light remitted from an in-vivo site through a color filter array with a predominance or larger number of blue elements to light in accordance with an embodiment of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE INVENTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well-known features may be omitted or simplified in order not to obscure the present invention.

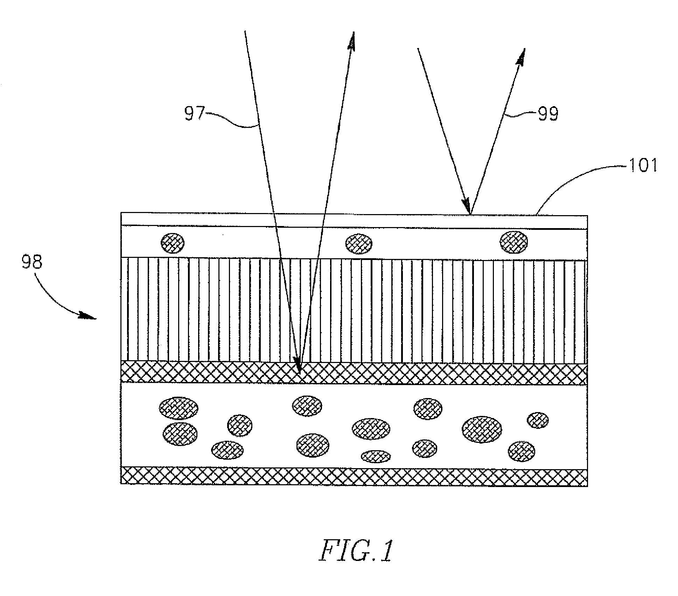

The absorption coefficient in tissue may be dominated by hemoglobin and melanin in the visible range of wavelengths. When considering the combined spectral response of red, blue, and green, red light may be considered to be almost transparent to the surface of the tissue while the blue and green light may only be partially absorbed by the tissue. The scattering coefficient may be inversely proportional to the wavelength of light. As such, red light may be less scattering than blue light and its effective penetration may be larger. Reference is now made to Fig. 1 showing schematically the penetration of blue wavelength 99 through a tissue 98 as compared to red wavelength 97. The red wavelength 97 may penetrate deeper into the tissue as compared to the blue wavelength 99. Therefore spatial features, especially from the surface of the tissue 101, may be sharpest in blue light being reflected directly off the surface of the tissue 101 as compared to green light and especially red light that may penetrate through the tissue before being reflected.

In some embodiments of the present invention, an imager may include a CFA with an increased number of blue sensitive elements, e.g. pixels that may be suitable, for example, for imaging surface details of, for example, a body lumen. Blue light, due to for example, its relatively short wavelength may be predominantly reflected from the surface of the tissue with little penetration into the tissue and therefore spatial features especially from the surface of the tissue may be sharper in blue light as compared to red and/or green light. In some embodiments of the present invention, the red plane may be flat as compared to the green and blue planes. Increasing the resolution of the blue plane as compared to the red plane may increase the contrast and spatial information that may be obtained from an image, for example, a color image, of an in-vivo tissue, e.g. the GI tract.

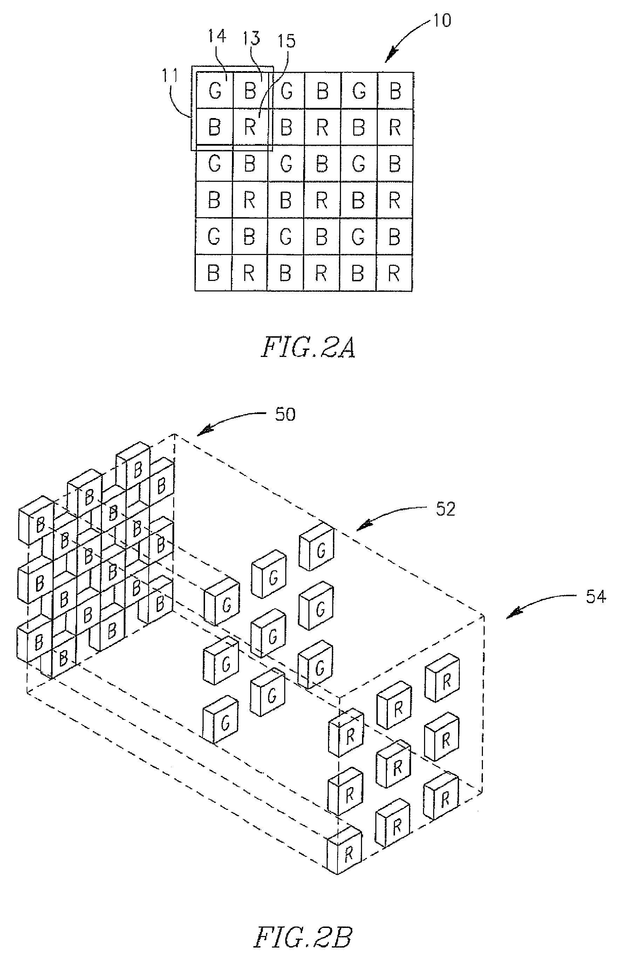

Reference is made to Fig. 2A and 2B, a schematic drawing of a color filter array pattern with color filter elements having a predominance or high frequency of blue elements in accordance with an embodiment of the invention. In some embodiments, a pattern of color elements in a color filter array 10 may include a repeating series, for example, of 2x2 block 11, where a blue filter element 13 may be alternated with a green filter element 14 and red filter element 15. In other embodiments, a blue filter element 13 may be alternated with filters other than and/or in addition to red and blue filters, for example, UV filter, IR filter, other visible color filter, and/or other non-visible filter. In some embodiments blue filter element 13, green filter element 14, and red filter element 15 may be arranged in other suitable patterns, for example, in other repeatable patterns, such that blue filter elements 13 appear more frequently than red filter elements 15 and/or more frequently than green filter elements 14, for example approximately twice as frequently as green filter elements 14 and approximately twice as frequently as red filtered elements 15. Other patterns may be possible, such as for example, 3x3 block, or other size and/or shape block including a repeatable pattern of color elements of, for example, blue, green, blue, red. In some embodiments blue filter elements 13 may be present with a frequency of less than every other element. Embodiments of the invention may include color patterns with a frequency of blue filter elements 13 of approximately two times that of red elements and green elements, such as for example between forty and fifty percent blue filter elements 13. Other numbers, percentages or arrangements of blue filter elements 13 may be used. Colors other than or in addition to red, blue and green may also be used. In some embodiments, green filter elements 14 and red filter elements 15 may be arranged in a repeating pattern with respect to the blue filter elements 13. As a result of the predominance of blue filter elements 13 in a filter array relative to the red and the green filter elements 14, details in an area being imaged that may reflect blue light may be sampled at a rate that may be approximately twice as frequent as the sampling of details that may reflect red and green light. Sampling reflected blue light with a higher resolution may increase the contrast and detail shown in the image of for example surface of a body lumen wall. Shadows and blurriness may be present when sampling colors with a relatively higher absorption coefficient, e.g. red filter elements 15.

In some embodiments, the individual colored elements of a color filter array 10 may be shaped as squares or other shapes. Other sizes and dimensions may be used.

Reference is made to Fig. 2B, an exploded view of a color filter array in accordance with an embodiment of the invention. In some embodiments, blue filter elements 50 may be interspersed alternately between green filter elements and red filter elements 54.

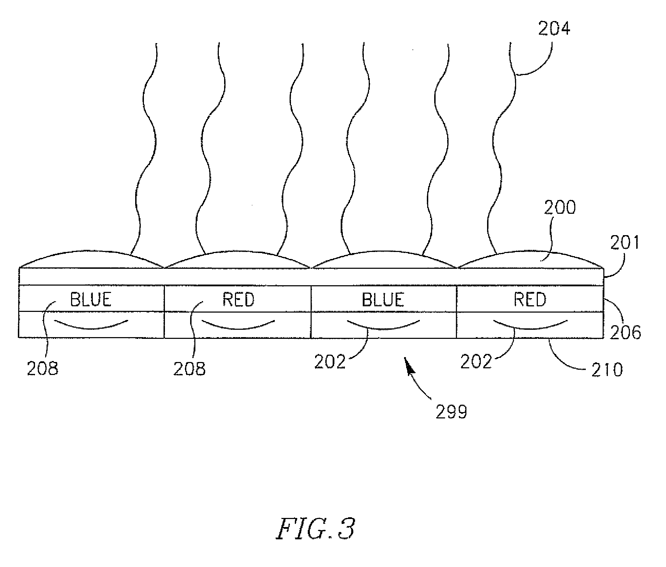

Fig. 3 is a schematic diagram of elements of a solid state imager having a color filter array in accordance with an embodiment of the invention. In some embodiments, imager 299 may include one or more micro-lenses 200 that may be formed over pixel cells or light sensitive elements 210. The micro-lens 200 may focus light 204 through the color filter element 208 and onto the photosensor 202 element in the pixel cell 210 below. In some embodiments a photosensor 202 may be or include one or more of a photodiode or photogate or other light sensors. In some embodiments, imager 299 may include a spacer layer 201 for example under the micro-lens 200 layer and over the color filter layer 206. The thickness of spacer layer 201 may be adjusted so that the photosensors 202 are at and/or near a focal point of light passing through micro-lens 200. A color filter layer 206 may be or include a substrate. Color filter layer 206 may be or include a color resist or acrylic material which may be used as a light transmitting material or filter. Other materials may be used and other suitable methods of isolating a specified frequency and/or spectrum of light may be used. The varying color filter elements 208 may be disposed side by side in the patterns described in respect of figure 1A and 1B. Other paints or dyes may be used to provide filtering characteristics to color filter elements 208. The individual color elements over filter layer 206 may be placed in registration with the photosensors 202 on the semiconductor layer of the imager. Pixel 210 cell array may include, for example, peripheral circuits and contacts and wiring formed thereon by known methods. A protective layer of for example silicon dioxide or silicon nitride may be formed over the pixel 210 layer to, for example, passivate it and to provide a planarized surface. Other suitable methods may be used to provide a planarized surface.

In some embodiments, the color filter array 10 of embodiments of the present invention may be or may be included in for example a charged couple device (CCD) or complementary metal oxide semi-conductor (CMOS) imager. Other imagers may be used. The imager may be combined, for example, with a processor, such as a CPU, digital signal processor or microprocessor. The imager and the microprocessor may be formed, for example, in a single integrated circuit. A processor based system is exemplary of a system having digital circuits which may include CMOS or other imager devices. Such a system may include for example a computer system, camera system, scanner, machine vision system, vehicle navigation system, video telephone, surveillance system, auto focus system, star tracker system, motion detection system, image stabilization system and data compression system for high-definition television, all of which may utilize embodiments of the invention.

Other in-vivo detecting and measuring systems, to which the imager having color filter layer 206 with color filter pattern 10 of embodiments of the present invention may be attached, are endoscopes. In a preferred embodiment of the present invention, color filter layer 206 may be included in or as a part of an in-vivo camera system or an in-vivo measurement system, which may detect images and analyze data of various in-vivo systems, such as for example the digestive, or other systems. In-vivo camera and measurement systems may include swallowable electronic devices such as capsules which collect data from various internal body organs or tissues and further transmit data to an external receiver system. These devices which may be for example swallowable intestinal capsules may further include a transmission system for transmitting the measured data at various radio frequencies to the receiver system.

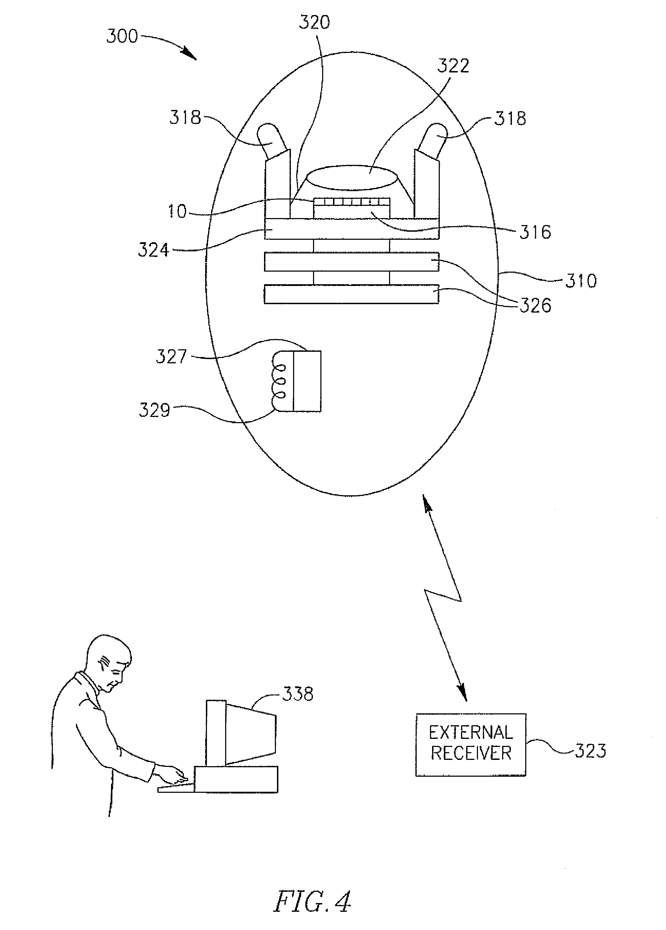

Reference is made to Fig. 4 showing a schematic diagram of a system including an imager with a color filter pattern in accordance with an embodiment of the invention. Imaging device 300 may include a sensing device such as for example an imager 316 within an outer housing 310 constructed and operative in accordance with an embodiment of the invention. Housing 310 may be, for example, spherical, ovoid, or any other suitable shape and may be partially deformable. Imager 316 may include for example a CCD or a CMOS imager. The imaging device 300 may include, for example an optical system such as for example a lens 322 and a lens holder 320, as well as one or more (e.g., a pair, a ring, etc.) illumination sources 318, such as light emitting diodes (LEDs), which may illuminate the areas to be imaged by the imaging sensor 316. In one embodiment of the present invention, illumination source 318 may include one or more white LED(s). In other embodiments of the present invention, illumination source 318 may include other sources of light, for example with different colors or wavelength ranges, e.g. UV light, IR light, etc. Imager 316 may include one or more color filter arrays 10. Positions for imager 316 other than on an end of housing 310 may be used. More than one illumination source 318 per imager may be used. Imaging device 300 may include a circuit board 324, one or more switches that may have a capacity to control or regulate one or more components in imaging device 300, and one or more power sources such as for example batteries 326. In some embodiments imaging device 300 may include a transmitter 327, such as for example a wireless or radio transmitter, and an antenna 329. In some embodiments of the present invention, transmitter 327 may be a transceiver and may receive signals from an external source. In other embodiments, a separate receiver may be included in imaging device 300. Imaging device 300 may transmit sensory data in the form of signals to an external receiver 323 where such signals or images may be stored or further processed for viewing on an external display 338 such as for example a monitor. In some embodiments, the transmitter 327 for example may transmit image signals to the external receiver 323 so that images may be viewed for example on-line. Other suitable viewing methods may be used. Transmitter 327 may include control capability for, for example controlling the various operations of imaging device 300, although control capability or one or more aspects of control may be included in a separate component. Transmitter 327 may typically be an ASIC (application specific integrated circuit), but may be of other constructions; for example, transmitter 327 may be a processor executing instructions. Imaging device 300 may include a processing unit separate from transmitter 327 that may, for example, contain or process instructions.

Imaging device 300 and/or outer housing 310 typically may be or may include an autonomous swallowable capsule, but imaging device 300 may have other shapes and need not be swallowable or autonomous. Embodiments of imaging device 300 are typically autonomous, and are typically self-contained. For example, imaging device 300 may be a capsule or other unit where all the components are substantially contained within a container or shell, and where imaging device 300 does not require any wires or cables to, for example, receive power or transmit information. Power may be provided to imaging device 300 by an internal battery or a wireless receiving system. Other embodiments may have other configurations and capabilities. For example, components may be distributed over multiple sites or units. Control information may be received from an external source.

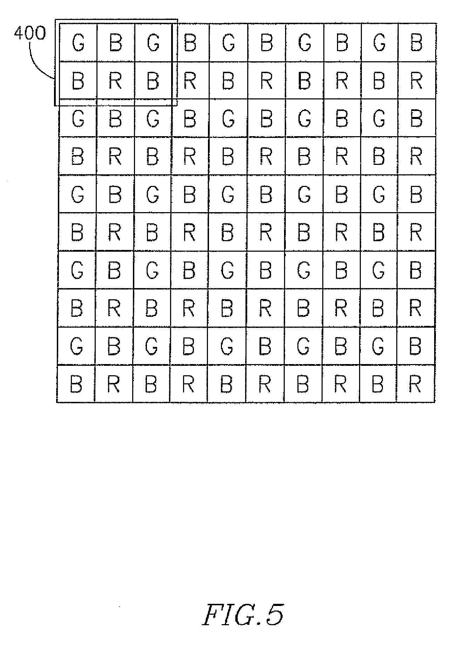

Embodiments of the present invention may be similar to or may include elements of or similar to embodiments described in US Application Publication No. 2001/0035902, entitled "A DEVICE AND SYSTEM FOR IN VIVO IMAGING" and published on November 1, 2001 as well as US Patent No. 5,604,531, entitled "AN IN VIVO VIDEO CAMERA SYSTEM", both of which are hereby incorporated by reference in their entirety. Reference is made to Fig. 5, a schematic drawing of a color filter array having alternate pattern of filter elements according to an embodiment of the invention. In some embodiments, the pattern may include, for example, a 3x3 block pattern 400 with for example five blue filter elements 13, two green filter elements 14, and two red filter element 15. Other patterns, other size blocks with other ratios between red, blue, and green, may be implemented. In one example, the filter elements may include filter elements other than red, blue, and green filter elements. In one embodiment of the present invention more than one color filter pattern may be included in an imaging device. For example, in some embodiments, a first color filter pattern, for example, positioned in the central imaging area of the imager may include a higher resolution of red and/or green sensitive elements and a second color filter pattern, for example, in the periphery may include a higher resolution of blue sensitive elements. Other arrangements of a first color pattern and a second color pattern may be used. Other numbers of different patterns may be used.

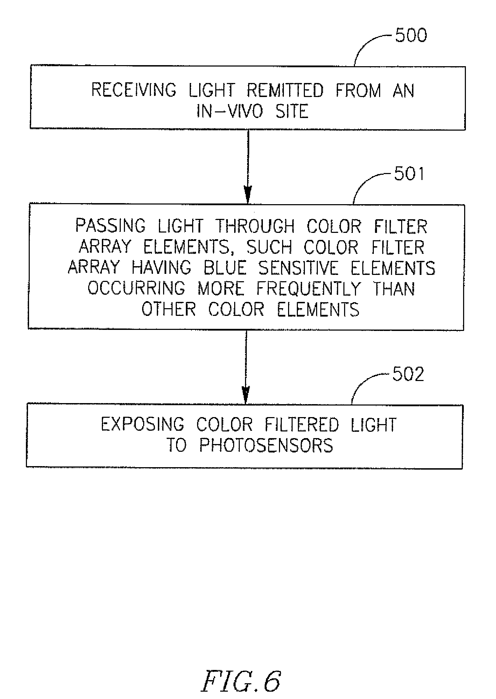

Reference is made to Fig. 6, showing a flow diagram of a method of receiving light remitted from an in-vivo site through a color filter array with a predominance or larger number of blue elements in accordance with an embodiment of the invention. In block 500, light remitted from an in-vivo site may be received. In block 501 light may be passed through elements of a color filter array. The color filter array may include for example red sensitive elements, e.g. a filter passing through red light, blue sensitive elements, e.g. a filter passing through blue light and green sensitive elements, e.g. a filter passing through green light arranged in a predefined pattern, such that the blue sensitive elements occur more frequently, for example, approximately twice as frequently in such pattern as the green sensitive elements and more frequently, for example, twice as frequently as the red sensitive elements. In block 502, a photosensor may be exposed to the light that passed through an element of the color filter array. In some embodiments, the color filter array may be included in a device that may be inserted (for example, by swallowing) into a gastro-intestinal tract. In some embodiments the color filter array may be part of a system that may capture an image of an in-vivo area. In other embodiments, a pattern of pixels receiving red, blue, or green light may be obtained by means other than a CFA. Other operations or series of operations may be used.

While the present invention has been described with reference to one or more specific embodiments, the description is intended to be illustrative as a whole and is not to be construed as limiting the invention to the embodiments shown. It is appreciated that various modifications may occur to those skilled in the art that, while not specifically shown herein, are nevertheless within the true spirit and scope of the invention.

## Claims

1. An in-vivo imaging device comprising a CFA, the CFA comprising red sensitive elements, blue sensitive elements, and green sensitive elements, the blue sensitive elements occurring in the CFA more frequently than the red sensitive elements and more frequently than the green sensitive elements.

2. The device of claim 1 wherein the blue sensitive elements occur approximately twice as frequently as the red sensitive elements and approximately twice as frequently as the green sensitive elements.

3. The device of claim 1, wherein the green sensitive elements and the red sensitive elements are arranged in a repeating pattern with respect to the blue sensitive elements.

4. The device of claim 1, wherein the red sensitive elements and the green sensitive elements alternate with the blue sensitive elements in alternate rows, in a repeating pattern.

5. The device of claim 1 wherein the CFA is included in a CMOS.

6. The device of claim 1, wherein the imaging device is a swallowable capsule.

7. A system comprising:
an in-vivo imaging device comprising:
an imager with a CFA, the CFA comprising red sensitive elements and blue sensitive elements, the blue sensitive elements occurring more frequently than the red sensitive elements; and
a transmitter for transmitting images captured by the imager to an external receiving unit; and
the external receiving unit.

8. The system of claim 7, wherein the blue sensitive elements occur approximately twice as frequently as the red sensitive elements.

9. The system of claim 7, wherein the imaging device comprises an optical system suitable for focusing light through the CFA.

10. The system of claim 7, wherein the imager is a CMOS.

11. The system of claim 7, wherein the imaging device comprises a power source.

12. The device of claim 7, wherein the imaging device is swallowable.

13. An imaging device comprising a CFA, the CFA comprising blue sensitive elements and at least one other color sensitive element, the blue sensitive elements occurring in the CFA more frequently than the at least one other color sensitive elements.

14. The device of claim 13, comprising red sensitive elements wherein the blue sensitive elements occur approximately twice as frequently as the red sensitive elements.

15. The device of claim 13, comprising green sensitive elements wherein the blue sensitive elements occur approximately twice as frequently as the green sensitive elements.

16. The device of claim 13 wherein the CFA is included in a CMOS.

17. The device of claim 13 wherein the CFA is included in a CCD.

18. The device of claim 13 wherein the imaging device is a swallowable capsule.
